# FASCICULE DE BREVET EUROPEEN

(11) **EP 1 741 451 B1**
(45) Date de publication et mention de la délivrance du brevet: **31.10.2012**
(21) Numéro de dépôt: 06113574.5
(22) Date de dépôt: 05.05.2006
(51) Int. Cl.: A61L 2/18, A01J 7/04

(54) **Utilisation pour le nettoyage et la desinfection dans le domaine de l'hygiène en production laitière**
Verwendung zur Reinigung und zur Desinfizierung im Bereich der Milchherstellungshygiene
Use for cleaning and desinfecting in the field of milk production hygiene

(30) Priorité: 09.05.2005 FR 0504614
(43) Date de publication de la demande: 10.01.2007
(73) Titulaire: Societe Hypred, 35803 Dinard Cedex (FR)
(72) Inventeur: Mourcel, Philippe, 35400, Saint-Malo (FR); Gougeon-Diederichs, Sophie, 35870, Le Minihic sur Rance (FR)
(74) Mandataire: Hart-Davis, Jason

(56) Documents cités:
- EP-A- 0 737 738
- US-A- 4 578 206
- US-A- 5 679 661
- PATENT ABSTRACTS OF JAPAN vol. 2000, no. 20, 10 juillet 2001 (2001-07-10) & JP 2001 072518 A (KAO CORP), 21 mars 2001 (2001-03-21)

## Description

La présente invention se rapporte au domaine du maintien de l'hygiène en production laitière. Elle a pour objet des utilisations, dont le but est d'assurer un nettoyage et une désinfection la plus complète possible de matériaux se trouvant au contact des trayons de vaches laitières. Il s'agit notamment du nettoyage et de la désinfection des lavettes appliquées aux trayons de vaches avant la traite et des faisceaux trayeurs permettant la traite mécanisée.

### Arrière-plan technologique

Les surfaces des trayons et des mamelles des vaches laitières sont facilement contaminées par des souillures diverses provenant des litières ou de l'environnement : déjections, débris de paille, poussières, terre, etc. Pour éviter d'introduire dans le lait ces souillures et en dégrader les qualités nutritives, organoleptiques ou bactériologiques, il est d'usage de procéder au nettoyage des trayons avant la traite. Cette procédure permet également de limiter la transmission de vache à vache de germes infectieux par l'intermédiaire du matériel de traite. Un nettoyage et/ou une désinfection insuffisante augmente les risques de mammites (transférées d'une vache à l'autre) et de taux cellulaires élevés dans le lait.

Le nettoyage des trayons est habituellement opéré en utilisant des lavettes. Dans la présente description et dans le cadre de la présente invention, on entend par « lavette », un matériau tissé ou non tissé pouvant être imprégné d'une solution d'un produit de nettoyage et/ou de désinfection. Les matériaux tissés les plus usuels utilisés dans la fabrication de lavettes destinées à être appliquées aux trayons de vaches sont à base de coton. Des lavettes non-tissées constituées d'agrégations de fibres collées entre elles sont également connues dans ce domaine.

Lors de leur utilisation pour nettoyer des trayons, les lavettes sont imprégnées par une solution de produit de nettoyage. Les lavettes peuvent être jetables et livrées à l'éleveur déjà imprégnées par une solution appropriée, ou bien, comme dans la plupart des cas, réutilisables - le recyclage des lavettes présente un intérêt économique évident. L'éleveur prépare alors dans un seau une solution par dilution d'un produit dans l'eau à la concentration préconisée par le fabricant, et y fait tremper ses lavettes avant de démarrer la traite. Outre le nettoyage de la peau, le massage du trayon a un effet stimulant et accélère la traite.

Les solutions de nettoyage utilisées contiennent essentiellement des agents lavants constitués de tensio-actifs anioniques, non ioniques ou amphotères. Ils peuvent également contenir des agents biocides destinés à éviter la prolifération bactérienne dans les solutions imprégnant les lavettes, par exemple par un effet bactériostatique ou bactéricide.

Parmi les tensio-actifs anioniques utilisés dans des solutions de nettoyage, on peut notamment citer: des alkylarylsulfonates (par exemple dodécylbenzène sulfonate de sodium, dodécylbenzène sulfonate de triéthanolamine, dodécylbenzène sulfonate d'isopropylamine) ; des alcanesulfonates secondaires dérivés de n-paraffines, comme HOSTAPUR^{®} SAS 60 commercialisé par la société CLARIANT ; des alphaoléfinesulfonates; des alkylsulfates (par exemple dodécylsulfate de sodium) ; des alkyléthersulfates dérivés d'alcools gras naturels ou synthétiques polyéthoxylés ; des savons de sodium ou de potassium.

Les tensio-actifs non ioniques sont souvent choisis parmi : les alcools gras polyéthoxylés, obtenus par réaction de l'oxyde d'éthylène sur un alcool gras en présence d'un catalyseur, le nombre d'unités d'oxyde d'éthylène ajoutées à l'alcool pouvant être très variable, de même que la nature de l'alcool gras : linéaire dérivé de corps gras d'origine végétale ou animale, linéaire obtenu par le procédé Ziegler, plus ou moins ramifié (alcools oxo dérivés d'alpha-oléfines, ou oligomères du propylène ou du butène) ; les oxydes d'alkylamines, par exemple oxyde de cocodiméthylamine ; les alcanolamides d'acides gras (par exemple diéthanolamide d'acides gras de coprah, monoéthanolamide d'acides gras de coprah) ; les alkylpolyglucosides.

Comme tensio-actifs amphotères, on peut notamment citer : les alkyldiméthylbétaïnes, les alkylamidopropylbétaïnes, des dérivés d'imidazoline (alkylamphoacétates, ...), les alkyliminodipropionates, les alkylpolyaminocarboxylates.

Les agents biocides, lorsqu'ils sont incorporés dans les produits de nettoyage des trayons, sont souvent des substances cationiques. On peut notamment citer : des dérivés de la guanidine, comme le chlorhydrate de poly(hexaméthylènebiguanide), les sels de chlorhexidine (par exemple digluconate de chlorhexidine), le chlorure de 1-coco alkyl guanidinium ; des alkyl(poly)amines, comme la laurylpropylènediamine ou la bis(3-aminopropyl)dodécylamine ; le chlorhydrate de décyloxy-3-hydroxy-2-amino-1-propane. Les composés d'ammonium quaternaire peuvent fonctionner dans ce rôle, mais ces composés ne sont pas préférés à cause des risques d'inhibition de la flore lactique en cas d'accident conduisant à la présence de résidus dans le lait.

D'autres biocides utilisables dans ce contexte comprennent notamment : le 2,4,4'-trichloro-2'-hydroxydiphényl-ether, connu sous le nom de TRICLOSAN, des composés amphotères (lauryl diéthylènetriaminoacétate, lauryl propylènediaminoacétate...). Le composé biocide doit être choisi pour être compatible avec les agents lavants, et être bien toléré par la peau de l'animal et de l'éleveur.

D'autres ingrédients peuvent être incorporés dans des compositions de nettoyage et de désinfection des trayons, tels que des émollients / hydratants, parfums, colorants, épaississants, etc.

Des exemples précis de compositions destinées au nettoyage des trayons et contenant au moins un agent biocide sont donnés par les brevets suivants : Dybas R. et al (Merck & Co), « Lower alkyl substituted diphenyl polyamine as an antimicrobial agent », US 4,311,709 ; Laroche B. et al, « Procédé pour le traitement des pis des mammifères, notamment des pis des vaches ainsi que composition pour la mise en oeuvre de ce procédé », FR 2 633 308 ; Zighelboim R.J., « Method of milking cows », US 5,366,732 ; Collin, A. et al, « Compositions destinées au traitement des trayons des mammifères avant et après la traite », EP 0 772 973 B1 ; Dyer D.L., « Compositions and methods for the treatment and prevention of bovine mastitis », US 6,525,071.

Il est courant dans ce domaine de diluer le produit tel qu'il est fourni par le fabricant à une concentration de 0,5 à 1% de sa concentration d'origine afin de parvenir à une solution de concentration appropriée pour le trempage des lavettes.

Comme il l'a été indiqué plus haut, pour des raisons économiques, les éleveurs dans le domaine laitier souhaitent recycler autant que possible les lavettes utilisées pour le nettoyage et la désinfection des trayons de vaches. Afin de parvenir à ce but, il est nécessaire de pouvoir éliminer toutes les souillures qui peuvent être transférées aux lavettes lorsque celles-ci, après avoir trempé dans la solution de nettoyage et de désinfection, sont effectivement appliquées aux trayons de vaches. Certaines souillures sont particulièrement difficiles à enlever, notamment les résidus de bouses de vaches constatés à certaines périodes de l'année, lorsque le régime alimentaire est modifié, comme par exemple à la mise à l'herbe au printemps. A ce moment, la digestion est moins complète et/ou entraîne des sécrétions plus importantes des glandes digestives et de l'intestin.

A ce jour la procédure suivie habituellement par beaucoup d'éleveurs est de conserver des lavettes ayant servi pour le nettoyage et la désinfection des trayons, notamment entre deux traites successives des vaches (tels que les traites du matin et du soir), dans le même type de composition que celle permettant le nettoyage et la désinfection des trayons. Ce procédé est naturellement simple d'application sur le terrain, car l'éleveur peut simplement préparer deux seaux, tous deux contenant une même composition de nettoyage et de désinfection des trayons, dont l'un servira pour tremper les lavettes avant de les appliquer aux trayons des vaches et l'autre servira pour recueillir les lavettes usagées en attendant la traite suivante.

On constate cependant que les compositions habituellement employées pour le nettoyage et la désinfection des trayons ne permettent pas de nettoyer et désinfecter en profondeur des lavettes, et celles-ci ne peuvent pas être recyclées autant de fois que le souhaiterait un éleveur pour des raisons économiques évidentes. Plus précisément, les lavettes, généralement blanches au départ, prennent rapidement à l'usage une teinte grise. De plus, la population bactérienne sur les lavettes n'est que faiblement diminuée, malgré la présence de biocides. La demanderesse postule, sans que ceci constitue une explication définitive ni limite la portée de la présente invention, que les biocides habituels s'adsorbent sur les fibres des lavettes avec pour conséquence une réduction de leur réelle efficacité désinfectante.

Le problème à résoudre dans le cadre de la présente invention a ainsi consisté à développer des compositions de nettoyage et de désinfection des lavettes qui permettent un nettoyage complet des lavettes ainsi qu'une désinfection satisfaisante. Des compositions permettant un nettoyage et une désinfection en profondeur des lavettes, au vu des souillures qu'il faut enlever dans le contexte de la production laitière, permettraient le recyclage de lavettes.

De surcroît, il est très préférable, non seulement pour des raisons économiques mais pour des raisons d'application pratique sur le terrain, de pouvoir obtenir un nettoyage et une désinfection complète à température ambiante, sans être obligé de chauffer.

Il est également bien entendu souhaitable de disposer de compositions qui, même aux concentrations faibles résiduelles après rinçage, ne provoqueront pas de réactions cutanées chez les vaches. Les compositions ne doivent pas en outre provoquer une dégradation rapide des lavettes utilisées.

Un autre problème dans le domaine de la production laitière concerne la désinfection des faisceaux trayeurs. En effet, et notamment lorsque l'on constate l'apparition de mammites chez des vaches, il est important de pouvoir désinfecter les faisceaux trayeurs entre leur application à deux vaches laitières conduites successivement à la traite. Le problème est analogue à celui du nettoyage et de la désinfection des lavettes dans la mesure où les souillures (portées par les trayons des vaches) sont les mêmes, la différence ici étant que la surface à nettoyer et à désinfecter n'est plus un matériau à base de fibres, mais une surface constituée de métaux, de matières plastiques et d'élastomères (caoutchoucs). La désinfection des faisceaux trayeurs est généralement effectuée par un trempage de quelques secondes à quelques minutes dans une solution appropriée.

### Résumé de l'invention

Il a maintenant été découvert que l'ensemble de problèmes associés au nettoyage et à la désinfection des lavettes peuvent être résolus de manière satisfaisante par l'utilisation de compositions comprenant :
a) un système alcalin donneur d'oxygène actif contenant au moins un composé libérateur d'oxygène actif ;
b) un activateur du composé libérateur d'oxygène actif ; et
c) un azurant optique.

Selon un premier aspect, l'invention porte ainsi sur les utilisations selon les revendications 1 et 2. L'invention se rapporte également à un procédé de nettoyage et/ou de désinfection de lavettes destinées au nettoyage de trayons de vache, comprenant les étapes de:
1) préparation de composition liquide contenant a) un système alcalin donneur d'oxygène actif contenant au moins un composé libérateur d'oxygène actif ; b) un activateur dudit composé libérateur d'oxygène actif ; et c) un azurant optique ;
2) la mise en contact d'au moins une lavette avec la composition préparée à l'étape 1) pendant une durée comprise entre 1 minute et 24 heures, à une température comprise entre 15 et 65°C.

L'invention se rapporte également à un procédé de nettoyage et/ou de désinfection de trayons de vache, comprenant les étapes de:
1) imprégnation d'au moins une lavette d'une composition de nettoyage et/ou de désinfection ; et
2) l'application de ladite lavette aux trayons de vache;
dans lequel la composition de nettoyage et/ou de désinfection comprend les trois constituants a), b) et c) énoncés ci-dessus.

L'invention se rapporte également à un procédé de nettoyage et/ou de désinfection d'un faisceau trayeur comprenant l'application audit faisceau trayeur d'une composition comme définie ci-dessus, de préférence par trempage desdits faisceaux trayeurs dans une composition comprenant les trois constituants a), b) et c) énoncés ci-dessus. La température pour le nettoyage et/ou la désinfection d'un faisceau trayeur sera de préférence choisie entre 15 et 65°C pour une durée comprise entre 5 secondes et 15 minutes. En effet, le temps de traitement sera ici bien moins important que pour une lavette, car le traitement d'un faisceau trayeur interviendra généralement en cours de traite entre deux vaches auxquelles un même faisceau trayeur sera appliqué, par exemple afin d'éviter que la mammite dont une première vache est atteinte ne soit transférée à la vache suivante.

De manière préférentielle, les compositions utilisées dans le cadre de la présente invention présentent les caractéristiques suivantes :
- le composé libérateur d'oxygène actif est présent à raison de 30 à 90%, et de préférence de 40 à 70%, en masse par rapport à la masse totale de l'ensemble des constituants solides de la composition, la teneur en oxygène actif qui en résulte étant comprise entre 4,0 et 13,0%, de préférence entre 5,0 et 10,0% ;
- le rapport de la quantité massique dudit activateur sur la quantité d'oxygène actif en termes massiques est comprise entre 0,8 et 3,0, de préférence entre 1,0 et 2,0 ; et
- le rapport de la quantité massique dudit azurant optique sur la quantité d'oxygène actif en termes massiques est comprise entre 0,01 et 0,05, de préférence entre 0,015 et 0,025.

La « teneur en oxygène actif » est définie comme la capacité oxydante, mesurée par une méthode adaptée, et exprimée en équivalent oxygène d'après la demi-réaction rédox suivante :

O + 2e⁻ => O²⁻

Une méthode courante pour mesurer l'oxygène actif consiste à oxyder en milieu acide les iodures, puis à titrer l'iode ainsi formé par le thiosulfate de sodium. Par exemple, le perborate de sodium tetrahydraté contient généralement entre 10 et 11% d'oxygène actif. Le même principe peut être utilisé pour définir la teneur en oxygène actif d'une composition, telles que les compositions utilisées dans le cadre de la présente invention préparées par mélange de différents constituants dont certains n'ont pas la qualité de libérateur d'oxygène actif.

Les compositions utilisées selon l'invention contiennent:
a) un système alcalin donneur d'oxygène actif contenant au moins un composé libérateur d'oxygène actif ;
b) un activateur du composé libérateur d'oxygène actif;
c) un azurant optique,
et présentent les caractéristiques suivantes :
- le composé libérateur d'oxygène actif est présent à raison de 30 à 90%, et de préférence de 40 à 70%, en masse par rapport à la masse totale de l'ensemble des constituants solides de la composition, la teneur en oxygène actif qui en résulte étant comprise entre 4,0 et 13,0%, de préférence entre 5,0 et 10,0% ;
- le rapport de la quantité massique dudit activateur sur la quantité d'oxygène actif en termes massiques est comprise entre 0,8 et 3,0, de préférence entre 1,0 et 2,0 ; et
- le rapport de la quantité massique dudit azurant optique sur la quantité d'oxygène actif en termes massiques est comprise entre 0,01 et 0,05, de préférence entre 0,015 et 0,025.

Les compositions utilisées selon la présente invention peuvent être préparées sous différentes formes solides, et notamment des poudres, des granulés et des comprimés. Afin de préparer de telles formes solides on pourra ajouter des excipients connus tels que des agents lubrifiants, des agents désintégrants.

### Description détaillée de l'invention

Le système alcalin donneur d'oxygène actif utilisé selon l'invention contient au moins un composé libérateur d'oxygène actif. Dans le cas où ce composé est lui-même alcalin, il n'est pas nécessaire de rajouter un composé alcalin. Dans le cas où le composé libérateur d'oxygène actif n'est pas de nature alcaline, au moins un composé alcalin sera rajouté afin de donner en solution à 10 g/L un pH ≥ 8,0, de préférence de 9,5 à 12,0.

Comme exemple de système alcalin donneur d'oxygène actif intrinsèquement alcalin, on peut citer le percarbonate de sodium (aussi appelé carbonate de sodium peroxyhydraté), un composé d'addition du carbonate de sodium et du peroxyde d'hydrogène, dont la formule brute peut être décrite comme Na₂CO₃.1,5H₂O₂.

Comme composé libérateur d'oxygène actif, moins alcalin que le percarbonate, on peut citer le perborate de sodium. Celui-ci est disponible commercialement sous forme de monohydrate ou de tétrahydrate (NaBO₃.H₂O et NaBO₃.4H₂O, respectivement). Lorsque le perborate est utilisé, il peut être avantageux d'ajouter un composé alcalin aux compositions.

Tout composé alcalin compatible avec les autres constituants des compositions peut être utilisé pour donner lieu à un pH alcalin en solution. On peut citer en particulier en tant que composés alcalins utilisables dans ce contexte : les carbonates (carbonate de sodium, sesquicarbonate de sodium) ; les silicates alcalins (métasilicate de sodium, disilicate de sodium...) ; les orthophosphates alcalins (orthophosphate trisodique...) ; les polyphosphates alcalins comme le pyrophosphate de sodium.

Dans un mode de réalisation préférentiel de la présente invention, on utilisera le percarbonate de sodium. Lorsque le percarbonate de sodium est utilisé, l'addition d'un sel alcalin supplémentaire n'est généralement pas nécessaire, puisque le carbonate de sodium obtenu après dissolution donne aux solutions un pH ≥10,0. Cependant, l'utilisation d'un composé alcalin supplémentaire dans une composition contenant le percarbonate de sodium n'est pas exclue.

L'activateur du composé libérateur d'oxygène actif dans le cadre de la présente invention peut être choisie parmi les substances communément dénommées comme suit: TAED (tétraacétyléthylènediamine) ; TAGU (tétraacétylglycolurile); PAG (pentaacétylglucose); DADHT (1,5-diacetyl-2,4-dioxohexahydro-1,3,5-triazine); SNOBS (nonanoyloxybenzènesulfonate de sodium); SLOBS (Iauroyloxybenzènesulfonate de sodium); acétylcaprolactame.

Un activateur préféré dans le cadre de la présente invention est la TAED (tétraacétyléthylènediamine). Les activateurs lipophiles, et en particulier le SNOBS et le SLOBS, peuvent aussi avantageusement être employés, notamment quand il est constaté que la souillure à traiter est de nature plutôt lipophile.

Il est intéressant de constater ici que ces activateurs sont généralement des agents d'acylation, et sont constitués d'une portion acyle sur laquelle est attaché un groupe libéré par la réaction d'acylation. Par exemple, en solutions alcalines, une molécule de TAED réagit avec 2 anions perhydroxyle pour donner une mole de diacétyléthylènediamine (DAED) et 2 anions peracétique :

TAED + 2 HOO⁻ => DAED + 2 CH₃COOO⁻

L'anion peracétique et l'acide peracétique sont en équilibre, le déplacement vers l'une ou l'autre forme dépendant du pH :

CH₃COOO⁻ + H⁺ ⇔ CH₃COOOH

L'acide peracétique est connu pour développer son activité désinfectante sous sa forme non dissociée, c'est à dire à un pH nettement inférieur à son pKa de 8,2 (par exemple, aux environs de pH 5 ou moins). En effet, sous sa forme non dissociée, donc non chargée électriquement, l'acide peracétique peut pénétrer les membranes cellulaires des microorganismes.

Cependant, de manière surprenante, les compositions utilisées selon l'invention manifestent une forte activité désinfectante pour des pH supérieurs ou égaux à 10,0 et à des températures faibles, de l'ordre de 20°C.

Les azurants optiques sont des substances chimiques possédant la propriété d'absorber la lumière ultraviolette et de la restituer sous forme de lumière bleue. Leur présence sur des substrats propres mais d'aspect jaunâtre renforce l'impression de blancheur.

Parmi les azurants optiques susceptibles d'être utilises dans le cadre de la présente invention, on peut citer notamment: 4,4'-bis[(4-anilino-6-morpholino-1,3,5-triazine-2-yl)amino]stilbène-2,2'-disulfonate de disodium, commercialisé par la société CIBA sous les marques TINOPAL^{®} DMS-X HAUTE CONC et TINOPAL^{®} DMA-X ; bis(phénylurée)-4,4'-stilbène-disulfonate-2,2' de sodium ; bis[(phénylamino-2) (diéthanolamine-6) triazinyl-1,3,5-amino-4]-4,4'-stilbène-disulfonate-2,2' de sodium ; (diméthylamino-3,5) (méthylcarboxylamide-6) [(sulfamyl-3)-phényl-carboxylamide]-2-pyrazine ; (parasulfamidophényl-1) (parachlorophényl-3) (dihydro-4,5)-pyrazole; alpha (benzimidazolyl-2) béta (N-hydroxyéthylbenzimidazolyl-2) éthylène.

Il a été constaté que dans les compositions utilisées selon l'invention, l'azurant optique avait une contribution plus importante qu'attendue ; il permet d'obtenir une blancheur des lavettes satisfaisante, alors qu'en son absence, on obtiendrait un aspect gris et terne.

Les compositions peuvent éventuellement contenir, en plus des composants décrits précédemment :
- des agents séquestrants, comme les polyphosphates, les gluconates, glucoheptonates, les citrates, les aminopolycarboxylates (EDTA...), des zéolites, permettant d'améliorer le lavage en eaux très dures et/ou d'augmenter la stabilité par la complexation des métaux. Selon un mode préférentiel de réalisation de la présente invention, on utilise un citrate en tant qu'agent séquestrant ;
- des agents anti-tartre fonctionnant par effet de seuil ou dispersion des cristaux de CaCO₃ formés en eaux dures ; il s'agit essentiellement de phosphonates, comme l'ATMP (acide aminotriméthylène phosphonique), le DTPMP (acide diéthylènetriaminepenta(méthylène phosphonique)), l'HEDP (acide 1-hydroxyethane-1,1-diphosphonique) ou le PBTC (acide phosphonobutanetricarboxylique ou acide phosphono-3-carboxyhexane-dioïque) ;
- des dispersants, comme les homopolymères ou copolymères de l'acide acrylique ou de l'acide maléique ou comme les bis-(poly-2-carboxyethyl)-phosphinates. Si un ou plusieurs dispersants est (sont) utilisé(s), la quantité totale de dispersant(s) est préférentiellement inférieure ou égale à 5% en poids par rapport au poids total des composants solides de la composition;
- des tensio-actifs anioniques, non ioniques, amphotères ou cationiques, par exemple choisis parmi ceux cités plus haut à titre d'arrière-plan technologique en rapport avec les solutions de nettoyage connues à ce jour dans le cadre de l'hygiène en production laitière; des mélanges de différents types de tensio-actifs sont possibles. Selon un mode préférentiel de réalisation de la présente invention, on utilise un tensio-actif anionique, non ionique ou un mélange des deux ;
- des adjuvants de fabrication, comme des lubrifiants pour la compression (tels que des polyéthylèneglycols, par exemple PEG-6000, ou le stéarate de calcium). Si un ou plusieurs adjuvant(s)s de fabrication est (sont) utilisé(s), la quantité totale d'adjuvant(s) de fabrication est préférentiellement inférieure ou égale à 10% en poids par rapport au poids total des composants solides de la composition ;
- des accélérateurs de dissolution, des agents désintégrants comme la polyvinylpyrrolidone réticulée, des amidons modifiés ou des gels de silice. Si un ou plusieurs accélérateur(s) de dissolution est (sont) utilisé(s), la quantité totale d'accélérateur(s) de dissolution est préférentiellement inférieure ou égale à 10% en poids par rapport au poids total des composants solides de la composition. Il est par ailleurs souhaitable que le poids de l'ensemble (adjuvant(s) de fabrication + accélérateur(s) de dissolution) ne dépasse pas 10% en poids par rapport au poids total des composants solides de la composition ;
- des charges minérales comme le sulfate de sodium ou le sulfate de magnésium, ou éventuellement le carbonate de calcium, les silices ou les chlorures tels que le chlorure de sodium. Pour éviter notamment une éventuelle influence négative sur la stabilité des préparations, selon un mode préférentiel de réalisation de la présente invention, on utilise un sulfate, et de préférence le sulfate de sodium ;
- des colorants. Tout colorant à la fois compatible avec les lavettes (ne colorant pas les lavettes) et dont la toxicité est suffisamment faible pour qu'il soit acceptable pour nettoyer des surfaces au contact de denrées alimentaires peut être utilisé. Des colorants alimentaires sont préférés dans ce contexte, par exemple E 131 Bleu Patenté V ou E 133 Bleu Brillant FCF. Si un ou plusieurs colorant(s) est (sont) utilisé(s), la quantité totale de colorant(s) est préférentiellement inférieure ou égale à 0,1% en poids par rapport au poids total des composants solides de la composition. Il est à noter que certains des autres composants des compositions selon la présente invention peuvent être commercialisés sous des formes comprenant déjà du colorant. C'est le cas par exemple de l'activateur TAED. L'utilisation de tels produits peut donc introduire une certaine quantité de colorant dans les compositions finales sans que du colorant supplémentaire y soit rajouté ;
- des parfums. Parmi les parfums qui peuvent être utilisés dans les compositions selon la présente invention, on peut citer des substances naturelles isolées ou en mélanges (huiles essentielles...), des substances parfumantes synthétiques, leurs mélanges, et des compositions parfumantes sous forme de mélanges de substances d'origine naturelle et de substances issues de la synthèse. Si un ou plusieurs parfum(s) est (sont) utilisé(s), la quantité totale de parfum(s) est préférentiellement inférieure ou égale à 1% en poids par rapport au poids total des composants solides de la composition ;

Les compositions utilisées selon l'invention contiennent de préférence une faible quantité de tensio-actif, entre au moins 0,1% et une quantité inférieure à 10% en poids par rapport au poids total des composants solides de la composition. Une teneur comprise entre 0,5% et 6% est encore plus préférable.

Les compositions utilisées selon l'invention renferment de préférence au moins un composant choisi parmi les séquestrants, les agents anti-tartre et les dispersants, fonctionnant par effet de seuil ou par dispersion des cristaux de CaCO₃. Lorsqu'un séquestrant est utilisé, il est présent à une concentration inférieure à 20% en poids par rapport au poids total des composants solides de la composition, et de préférence comprise entre 0,1% et 12%. Lorsque un agent anti-tartre est utilisé, il est présent à une concentration inférieure à 2% en poids par rapport au poids total des composants solides de la composition, de préférence entre 0,1 et 1%. Bien que ce ne soit pas nécessaire pour atteindre les objectifs de l'invention, il est possible d'associer un séquestrant et un agent anti-tartre. De préférence, on utilisera seulement un agent séquestrant sans agent anti-tartre ou dispersant, et encore plus préférentiellement l'agent séquestrant choisi sera un citrate.

Les compositions utilisées selon l'nvention contiennent de préférence une charge minérale, qui est de préférence présente à une concentration inférieure à 30% en poids par rapport au poids total des composants solides de la composition, et de préférence comprise entre 15% et 26%.

Selon un mode préférentiel de réalisation de la présente invention, celle-ci se rapporte donc à l'utilisation d'une composition solide destinée à la production de compositions liquides pour le maintien de l'hygiène en production laitière constituée essentiellement de :
a) un système alcalin donneur d'oxygène actif contenant au moins un composé libérateur d'oxygène actif ;
b) un ou des activateur(s) du composé libérateur d'oxygène actif ;
c) un ou des azurant(s) optique(s) ;
d) un ou des tensioactif(s) ;
e) un ou des séquestrant(s) et/ou un ou des agent(s) anti-tartre ; et
f) une ou des charge(s) minérale(s) ;
dans laquelle :
- le composé libérateur d'oxygène actif est présent à raison de 30 à 90%, et de préférence de 40 à 70%, en masse par rapport à la masse totale de l'ensemble des constituants solides de la composition, la teneur en oxygène actif qui en résulte étant comprise entre 4,0 et 13,0%, de préférence entre 5,0 et 10,0% ;
- le rapport de la quantité massique dudit activateur (ou desdits activateurs) sur la quantité d'oxygène actif en termes massiques est comprise entre 0,8 et 3,0, de préférence entre 1,0 et 2,0 ;
- le rapport de la quantité massique dudit azurant optique (ou desdits azurants optiques) sur la quantité d'oxygène actif en termes massiques est comprise entre 0,01 et 0,05, de préférence entre 0,015 et 0,025 ;
- le(s) tensioactif(s) est (sont) présent(s) à raison de 0,1 à 10%, et de préférence de 0,5 à 6%, en masse par rapport à la masse totale de l'ensemble des constituants solides de la composition ;
- le(s) séquestrant(s), si présent(s), constitue(nt) moins de 20%, et de préférence de 0,1 à 12%, en masse par rapport à la masse totale de l'ensemble des constituants solides de la composition, et l'agent(s) anti-tartre, si présent(s), constitue(nt) moins de 2%, et de préférence de 0,1 à 1%, en masse par rapport à la masse totale de l'ensemble des constituants solides de la composition ; et
- le(s) charge(s) minérale(s) constitue(nt) moins de 30 %, et de préférence de 15 à 26 %, en masse par rapport à la masse totale de l'ensemble des constituants solides de la composition.

Par l'expression « constituée essentiellement de », on entend signifier que des composants supplémentaires éventuels dans les compositions solides, de par leur nature et par leur concentration, n'affectent pas de manière significative le fonctionnement technique des compositions selon l'invention et la résolution du problème posé. Par exemple, la présence de quantités minimales de parfums ou de colorants n'aura pas d'effet matériel sur la performance des compositions selon l'invention dans le nettoyage et/ou dans la désinfection de lavettes ou de faisceaux trayeurs. De manière avantageuse, on n'utilisera pas, ou on réduira au minimum, la quantité de colorant(s) et de parfum(s). Les colorants peuvent notamment présenter l'inconvénient de colorer de manière inappropriée des lavettes, tandis que les parfums peuvent être nuisibles dans ce domaine où ils pourraient se retrouver dans des produits laitiers, et on peut aussi noter la possibilité que certains parfums provoquent des réactions allergiques chez les utilisateurs de compositions selon l'invention. Cependant, bien que des compositions sans colorant ni parfum soient particulièrement avantageuses dans le cadre de la présente invention, la présence de colorants et/ou de parfums dans les sources commerciales des composants à mélanger peut dans la pratique rendre difficile l'exclusion totale de colorants et de parfums.

Selon un mode encore plus préférentiel de réalisation de la présente invention, celle-ci se rapporte à l'utilisation d'une composition solide destinée à la production de compositions liquides pour le maintien de l'hygiène en production laitière constituée essentiellement de :
a) un système alcalin donneur d'oxygène actif contenant au moins un composé libérateur d'oxygène actif, le composé libérateur d'oxygène actif étant un percarbonate de sodium ou un perborate de sodium;
b) la TAED (tétraacétyléthylènediamine) en tant qu'activateur du composé libérateur d'oxygène actif;
c) un ou des azurant(s) optique(s) ;
d) un ou des tensioactif(s) choisi parmi les tensioactifs anioniques ou non ioniques ou un mélange des deux;
e) un citrate en tant qu'agent séquestrant; et
f) le sulfate de sodium en tant que charge minérale;
dans laquelle :
- le composé libérateur d'oxygène actif est présent à raison de 40 à 70% en masse par rapport à la masse totale de l'ensemble des constituants solides de la composition, la teneur en oxygène actif qui en résulte étant comprise entre 5,0 et 10,0% ;
- le rapport de la quantité massique dudit activateur TAED sur la quantité d'oxygène actif en termes massiques est comprise entre 1,0 et 2,0 ;
- le rapport de la quantité massique dudit azurant optique (ou desdits azurants optiques) sur la quantité d'oxygène actif en termes massiques est comprise entre 0,015 et 0,025 ;
- le(s) tensioactif(s) est (sont) présent(s) à raison de 0,1 à 10% en masse par rapport à la masse totale de l'ensemble des constituants solides de la composition ;
- le séquestrant (citrate) constitue de 0,1 à 12% en masse par rapport à la masse totale de l'ensemble des constituants solides de la composition; et
- la charge minérale (sulfate de sodium) constitue de 15 à 26 % en masse par rapport à la masse totale de l'ensemble des constituants solides de la composition.

Bien entendu, la présence de quantités minimales d'autres agents, tels que des parfums ou des colorants, ou des agents anti-tartre ou dispersants à des teneurs inférieures à celle du citrate, n'aura pas d'effet matériel sur le fonctionnement de la présente invention.

Selon un mode de réalisation particulièrement préférentiel de la présente invention, celle-ci se rapporte à l'utilisation des compositions solides contenant un nombre restreint de composants, sans colorant ni parfum, et de telles compositions solides destinées à la production de compositions liquides pour le maintien de l'hygiène en production laitière sont constituées de :
a) un système alcalin donneur d'oxygène actif contenant au moins un composé libérateur d'oxygène actif, le composé libérateur d'oxygène actif étant un percarbonate de sodium ou un perborate de sodium;
b) la TAED (tétraacétyléthylènediamine) en tant qu'activateur du composé libérateur d'oxygène actif;
c) un ou des azurant(s) optique(s) ;
d) un ou des tensioactif(s) choisi parmi les tensioactifs anioniques ou non ioniques ou un mélange des deux;
e) un citrate en tant qu'agent séquestrant; et
f) le sulfate de sodium en tant que charge minérale ;
dans laquelle :
- le composé libérateur d'oxygène actif est présent à raison de 40 à 70% en masse par rapport à la masse totale de l'ensemble des constituants solides de la composition, la teneur en oxygène actif qui en résulte étant comprise entre 5,0 et 10,0% ;
- le rapport de la quantité massique dudit activateur TAED sur la quantité d'oxygène actif en termes massiques est comprise entre 1,0 et 2,0 ;
- le rapport de la quantité massique dudit azurant optique (ou desdits azurants optiques) sur la quantité d'oxygène actif en termes massiques est comprise entre 0,015 et 0,025 ;
- le(s) tensioactif(s) est (sont) présent(s) à raison de 0,1 à 10% en masse par rapport à la masse totale de l'ensemble des constituants solides de la composition ;
- le séquestrant (citrate) constitue de 0,1 à 12% en masse par rapport à la masse totale de l'ensemble des constituants solides de la composition; et
- la charge minérale (sulfate de sodium) constitue de 15 à 26 % en masse par rapport à la masse totale de l'ensemble des constituants solides de la composition.

Les compositions utilisées selon l'invention se présentent généralement sous une forme solide, qui peut être une poudre, des granulés, des comprimés ou des blocs. Elles peuvent éventuellement se présenter sous forme de dispersions visqueuses ou de gels, à condition que le diluant utilisé ne déstabilise pas les autres composants.

Pour la réalisation de compositions utilisées selon l'invention sous forme de comprimés, on fait appel à des adjuvants de fabrication et/ou des accélérateurs de dissolution. La présente invention se rapporte donc également à l'utilisation des comprimés destinés à la production de compositions liquides pour le maintien de l'hygiène en production laitière, un tel comprimé étant constitué essentiellement de :
a) un système alcalin donneur d'oxygène actif contenant au moins un composé libérateur d'oxygène actif ;
b) un ou des activateur(s) du composé libérateur d'oxygène actif ;
c) un ou des azurant(s) optique(s) ;
d) un ou des tensioactif(s) ;
e) un ou des séquestrant(s) et/ou un ou des agent(s) anti-tartre ;
f) une ou des charge(s) minérale(s) ; et
g) un ou des adjuvant(s) de fabrication et/ou un ou des accélérateur(s) de dissolution ;
dans lequel :
- le composé libérateur d'oxygène actif est présent à raison de 30 à 85%, et de préférence de 40 à 65%, en masse par rapport à la masse totale de l'ensemble des constituants solides de la composition, la teneur en oxygène actif qui en résulte étant comprise entre 4,0 et 12,5%, de préférence entre 5,0 et 9,5% ;
- le rapport de la quantité massique dudit activateur (ou desdits activateurs) sur la quantité d'oxygène actif en termes massiques est comprise entre 0,8 et 3,0, de préférence entre 1,0 et 2,0 ;
- le rapport de la quantité massique dudit azurant optique (ou desdits azurants optiques) sur la quantité d'oxygène actif en termes massiques est comprise entre 0,01 et 0,05, de préférence entre 0,015 et 0,025 ;
- le(s) tensioactif(s) est (sont) présent(s) à raison de 0,1 à 10%, et de préférence de 0,5 à 5%, en masse par rapport à la masse totale de l'ensemble des constituants solides de la composition ;
- le(s) séquestrant(s), si présent(s), constitue(nt) moins de 20%, et de préférence de 0,1 à 12%, en masse par rapport à la masse totale de l'ensemble des constituants solides de la composition, et l'agent(s) anti-tartre, si présent(s), constitue(nt) moins de 2%, et de préférence de 0,1 à 1%, en masse par rapport à la masse totale de l'ensemble des constituants solides de la composition ;
- le(s) charge(s) minérale(s) constitue(nt) moins de 30 %, et de préférence de 5 à 25 %, en masse par rapport à la masse totale de l'ensemble des constituants solides de la composition ; et
- le ou les adjuvant(s) de fabrication constitue(nt) moins de 10%, et de préférence de 0,1 à 6%, en masse par rapport à la masse totale de l'ensemble des constituants solides de la composition ; et le ou les accélérateur(s) de dissolution constitue(nt) moins de 10%, et de préférence de 0,5 à 6%, en masse par rapport à la masse totale de l'ensemble des constituants solides de la composition. De préférence, le poids de l'ensemble (adjuvant(s) de fabrication + accélérateur(s) de dissolution) ne dépassera pas 10% en poids par rapport au poids total des composants solides de la composition.

Bien entendu, la présence de quantités minimales de parfums ou de colorants n'aura pas d'effet matériel sur la performance des comprimés selon l'invention.

Selon un mode préférentiel de réalisation de la présente invention, celle-ci se rapporte à l'utilisation des comprimés destinés à la production de compositions liquides pour le maintien de l'hygiène en production laitière, un tel comprimé étant constitué essentiellement de :
a) un système alcalin donneur d'oxygène actif contenant au moins un composé libérateur d'oxygène actif, le composé libérateur d'oxygène actif étant un percarbonate de sodium ou un perborate de sodium;
b) la TAED (tétraacétyléthylènediamine) en tant qu'activateur du composé libérateur d'oxygène actif;
c) un ou des azurant(s) optique(s) ;
d) un ou des tensioactif(s) choisi parmi les tensioactifs anioniques ou non ioniques ou un mélange des deux;
e) un citrate en tant qu'agent séquestrant; et
f) le sulfate de sodium en tant que charge minérale ; et
g) un ou des adjuvant(s) de fabrication et/ou un ou des accélérateur(s) de dissolution ;
dans lequel :
- le composé libérateur d'oxygène actif est présent à raison de 40 à 65% en masse par rapport à la masse totale de l'ensemble des constituants solides de la composition, la teneur en oxygène actif qui en résulte étant comprise entre 5,0 et 9,5% ;
- le rapport de la quantité massique dudit activateur TAED sur la quantité d'oxygène actif en termes massiques est comprise entre 1,0 et 2,0 ;
- le rapport de la quantité massique dudit azurant optique (ou desdits azurants optiques) sur la quantité d'oxygène actif en termes massiques est comprise entre 0,015 et 0,025 ;
- le(s) tensioactif(s) est (sont) présent(s) à raison de 0,1 à 10% en masse par rapport à la masse totale de l'ensemble des constituants solides de la composition ;
- le séquestrant (citrate) constitue de 0,1 à 12% en masse par rapport à la masse totale de l'ensemble des constituants solides de la composition;
- la charge minérale (sulfate de sodium) constitue de 5 à 25 % en masse par rapport à la masse totale de l'ensemble des constituants solides de la composition ; et
- le ou les adjuvant(s) de fabrication constitue(nt) moins de 10%, et de préférence de 0,1 à 6%, en masse par rapport à la masse totale de l'ensemble des constituants solides de la composition ; et le ou les accélérateur(s) de dissolution constitue(nt) moins de 10%, et de préférence de 0,5 à 6%, en masse par rapport à la masse totale de l'ensemble des constituants solides de la composition. De préférence, le poids de l'ensemble (adjuvant(s) de fabrication + accélérateur(s) de dissolution) ne dépassera pas 10% en poids par rapport au poids total des composants solides de la composition.

Bien entendu, la présence de quantités minimales d'autres agents, tel que des parfums ou des colorants, ou des agents anti-tartre ou dispersants à des teneurs inférieures à celle du citrate, n'aura pas d'effet matériel sur le fonctionnement de la présente invention.

Selon un autre mode de réalisation particulièrement préférentiel de la présente invention, celle-ci se rapporte à l'utilisation des comprimés contenant un nombre restreint de composants, sans colorant ni parfum, un tel comprimé destiné à la production de compositions liquides pour le maintien de l'hygiène en production laitière étant constitué de :
a) un système alcalin donneur d'oxygène actif contenant au moins un composé libérateur d'oxygène actif, le composé libérateur d'oxygène actif étant un percarbonate de sodium ou un perborate de sodium;
b) la TAED (tétraacétyléthylènediamine) en tant qu'activateur du composé libérateur d'oxygène actif;
c) un ou des azurant(s) optique(s) ;
d) un ou des tensioactif(s) choisi parmi les tensioactifs anioniques ou non ioniques ou un mélange des deux;
e) un citrate en tant qu'agent séquestrant;
f) le sulfate de sodium en tant que charge minérale ;
g) un polyethylèneglycol en tant qu'agent pour la compression (adjuvant d'aide à la fabrication) et/ou une polyvinylpyrrolidone réticulée ou non et/ou un gel de silice en tant qu'accélérateur de dissolution;
dans lequel :
- le composé libérateur d'oxygène actif est présent à raison de 40 à 65% en masse par rapport à la masse totale de l'ensemble des constituants solides de la composition, la teneur en oxygène actif qui en résulte étant comprise entre 5,0 et 9,5% ;
- le rapport de la quantité massique dudit activateur TAED sur la quantité d'oxygène actif en termes massiques est comprise entre 1,0 et 2,0 ;
- le rapport de la quantité massique dudit azurant optique (ou desdits azurants optiques) sur la quantité d'oxygène actif en termes massiques est comprise entre 0,015 et 0,025 ;
- le(s) tensioactif(s) est (sont) présent(s) à raison de 0,1 à 10% en masse par rapport à la masse totale de l'ensemble des constituants solides de la composition ;
- le séquestrant (citrate) constitue de 0,1 à 12% en masse par rapport à la masse totale de l'ensemble des constituants solides de la composition;
- la charge minérale (sulfate de sodium) constitue de 5 à 25 % en masse par rapport à la masse totale de l'ensemble des constituants solides de la composition; et
- l'adjuvant de fabrication (polyethylèneglycol) constitue moins de 10%, et de préférence de 0,1 à 6%, en masse par rapport à la masse totale de l'ensemble des constituants solides de la composition ; le ou les accélérateur(s) de dissolution (polyvinylpyrrolidone et/ou gel de silice) constitue(nt) moins de 10%, et de préférence de 0,5 à 6%, en masse par rapport à la masse totale de l'ensemble des constituants solides de la composition, et le poids de l'ensemble (polyethylèneglycol + polyvinylpyrrolidone + gel de silice) ne dépasse pas 10% en poids par rapport au poids total des composants solides de la composition.

La présence en forte concentration d'un composé libérateur d'oxygène actif et d'un activateur n'est a priori pas favorable à la stabilité du composé libérateur d'oxygène actif. En effet, même en présence de faibles quantités d'humidité libre tel qu'il résulte du mélange à sec des différents composants, on peut craindre un début de réaction entre composé libérateur d'oxygène actif et activateur, conduisant à la formation d'eau supplémentaire et à une accélération de la réaction de décomposition. En présence d'humidité, H₂O₂ libéré par le composé libérateur d'oxygène actif se décompose progressivement selon :

H₂O₂ => H₂O + ½ O₂

Cette réaction est accélérée par la température, et est catalysée par les métaux présents à l'état de traces dans la composition, en particulier par les métaux de transition. L'oxygène libéré sous forme gazeuse risque d'augmenter la pression interne des emballages, par exemple lorsque les compositions sont conditionnées dans des sachets étanches.

De manière surprenante, les compositions utilisées selon l'invention, bien que fortement concentrées en agents actifs, restent stables, même lorsque l'agent libérateur d'oxygène actif utilisé est le percarbonate de sodium, plus sensible à la décomposition. Cette stabilité est obtenue même en l'absence d'adjuvants permettant de lier l'eau par la formation d'hydrates, comme le tripolyphosphate de sodium.

De même, il a été découvert que les compositions utilisées selon l'invention pouvait être directement mises en forme de « tablettes » par compression, sans nécessité d'addition d'adjuvants en quantité significative, donc sans dilution significative de la formule. Par exemple, les quantités d'agents lubrifiants et d'agents de désintégration peuvent être très faibles, inférieurs à 10% de la masse totale de la composition, et même inférieurs à 5%, tout en permettant :
- la fabrication de comprimés à une cadence élevée sans collage du produit sur les poinçons
- la fabrication de comprimés de grande taille ; des comprimés de 30 à 50 g peuvent en effet être obtenus sans difficulté
- une dissolution rapide.

### EXEMPLES

Les dénominations des composants utilisés sont précisées ci-dessous :
- NaBO₃.4 H₂O: Perborate de sodium tétrahydrate
- Na₂CO₃.1,5 H₂O₂: Percarbonate de sodium OXYPER^{®} (SOLVAY)
- TAED: MYKON® ATC bleu (WARWICK)
- Azurant optique: TINOPAL® DMS-X HAUTE CONC (CIBA)
- DDBSNa 85%: Dodécylbenzène sulfonate de Na, NANSA^{®} HS 85/S (HUNTSMAN)
- Citrate.3Na 2H₂O: Citrate trisodique dihydrate
- DTPMP.Na₇: Sel de sodium, MASQUOL^{®} P550 Na P (SYNTHRON)
- HEDP. Na₄: CUBLEN^{®} K8514 P (ZSCHIMMER & SCHWARZ)
- PBTC.Na₄: BAYHIBIT^{®} S (LANXESS)
- Na₂SO₄: Sulfate de sodium anhydre
- SYLOID 244 FP: Gel de silice amorphe (GRACE DAVISON)
- KOLLIDON CL: Polyvinylpolypyrrolidone ou Polivinylpyrrolidone réticulée (BASF)
- POLYPLASDONE XL: Polyvinylpolypyrrolidone ou Polivinylpyrrolidone réticulée (ISP)
- DISINTEX 200: Polyvinylpolypyrrolidone ou Polivinylpyrrolidone réticulée (ISP)

Tous les pourcentages indiqués sont exprimés en masse, sauf indication contraire.

### Exemples comparatifs 1 à 4

Les compositions A à D ont été fabriquées par mélange à sec, puis comparées pour leur efficacité à nettoyer des lavettes. Pour cela, un lot homogène de lavettes blanches en synthétique a été prélevé en élevage puis trempé dans des solutions des différentes compositions. Au début de l'essai, la température des solutions était de 40°C . Les lavettes ont été conservées dans les solutions pendant environ 5 heures, sans maintien de la température ; la température des solutions a donc diminué rapidement, et était très proche de la température ambiante à la fin de l'essai.

La concentration minimale permettant d'éliminer les souillures visibles est reportée, et la blancheur évaluée par l'opérateur.

| Exemple comparatif | 1 | 2 | 3 | 4 |
|---|---|---|---|---|
| | A | B | C | D |
| NaB0₃.4H₂O | 100,0 % | 50,0 % | / | / |
| Na₂CO₃.1,5H₂O₂ | / | / | 50,0 % | 50,0 % |
| TAED | / | 10,0 % | 10,0 % | 10,0 % |
| DDBSNa 85% | / | / | / | 5,0 % |
| Citrate.3Na 2H₂O | / | / | / | 10,0 % |
| Na₂SO₄ | / | 40,0 % | 40,0 % | 25,0 % |
| | | | | |
| Oxygène actif | 10,5 % | 5,2 % | 6,8 % | 6,8 % |
| | | | | |
| Concentration minimale pour nettoyage | ≥ 50 g/L | 10 - 20 g/L | 10 - 20 g/L | 5 -10 g/L |
| Odeur | / | / | / | Satisfaisante |
| Blancheur | Non satisfaisante | Non satisfaisante | Non satisfaisante | Non satisfaisante |

La formule D a été utilisée pendant 10 jours à 10 g/L dans une exploitation de 40 vaches laitières. Les lavettes ont été systématiquement trempées pendant la durée des périodes inter traite, à une température initiale de 40°C environ.

Après 10 jours, les lavettes apparaissaient toujours propres et sans odeur, mais le blanchiment n'était pas satisfaisant, avec un aspect uniformément terne et grisâtre. La répétition du traitement n'a pas permis de récupérer l'aspect des lavettes neuves.

### Exemples comparatifs 5 à 7 et exemple 1

L'aspect terne et grisâtre des textiles est normalement associé au lavage en eau dure et entartrante, avec le dépôt sur les fibres de cristaux de carbonate de calcium et autres incrustations provoquées par la dureté de l'eau. Ces dépôts minéraux sur les fibres emprisonnent des particules de souillures, donnant la couleur grisâtre aux fibres.

Les compositions E à G ont alors été préparées, différant de la composition D par l'incorporation d'agents anti-tartre à effet de seuil, permettant à très faible concentration d'éviter la formation d'incrustations minérales sur les surfaces.

Même si l'aspect grisâtre des lavettes ne pouvait a priori pas être amélioré de manière significative par l'addition d'un azurant optique, une composition H (exemple 1) incorporant 0,2% de TINOPAL® DMS-X a été incluse dans l'essai.

Les compositions ont été testées selon la procédure indiquée plus haut pour les compositions A à D (exemples comparatifs 1 à 4), sur des lavettes en coton, à une concentration constante de 10 g/L, en comparaison avec la composition D.

| | Exemple comparatif 4 | Exemple comparatif 5 | Exemple comparatif 6 | Exemple comparatif 7 | Exemple 1 |
|---|---|---|---|---|---|
| | D | E | F | G | H |
| Na₂CO₃.1,5 H₂O₂ | 50,0 % | 50,0 % | 50,0 % | 50,0 % | 50,0 % |
| TAED | 10,0 % | 10,0 % | 10,0 % | 10,0 % | 10,0 % |
| DDBSNa 85% | 5,0 % | 5,0 % | 5,0 % | 5,0 % | 5,0 % |
| Citrate.3Na 2H₂O | 10,0 % | 10,0 % | 10,0 % | 10,0 % | 10,0 % |
| Na₂SO₄ | 25,0 % | 24,0 % | 24,0 % | 24,0 % | 24,8% |
| DTPMP.Na₇ | / | 1,0 % | / | / | / |
| HEDP. Na₄ | / | / | 1,0 % | / | / |
| PBTC.Na₄ | / | / | / | 1,0 % | / |
| Azurant optique | / | / | / | / | 0,2 % |
| | | | | | |
| Nettoyage | Satisfaisant | Satisfaisant | Satisfaisant | Moyen | Satisfaisant |
| Blancheur | Non satisfaisante | Moyen | Moyen | Non satisfaisante | Satisfaisant |

Les compositions E et F apportent une légère amélioration par rapport à la composition D. De manière surprenante, cette amélioration est faible comparée à celle procurée par l'azurant optique dans la composition H objet de l'invention.

Il est à noter que, probablement, une partie de l'efficacité nettoyante des compositions de l'invention provient de l'action mécanique ; en effet, ces compositions sont légèrement effervescentes à la dissolution dans l'eau.

### Propriétés de la composition selon l'exemple 1 de l'invention

### Stabilité de la composition solide

Un lot de la composition H a été fabriqué avec une teneur initiale en oxygène actif de 6,65%, et maintenue à 30°C dans un emballage plastique étanche. Après 2 mois, la teneur en oxygène actif était encore de 6,25 %, soit 94,0% de la valeur initiale.

Malgré les concentrations élevées en percarbonate de sodium et TAED, et la présence dans la composition de citrate de sodium sous forme hydratée, la stabilité était tout à fait satisfaisante.

### Activité bactéricide

La composition H a été testée pour son activité bactéricide selon la procédure de la norme EN 1276, dans les conditions de saleté. Les concentrations bactéricides, réduisant la population bactérienne d'au moins 5 log, sont indiquées ci-dessous en % masse / volume :

| | Température | |
|---|---|---|
| Temps de contact | 20°C | 40°C |
| 15 min | / | 2 % |
| 5 h | 0,25 % | 0,25 % |

Les compositions objet de l'invention présentent une forte activité désinfectante, même si l'acide peracétique produit in situ se trouve à un pH fortement alcalin (le pH à 1% masse / volume de la préparation H est d'environ 10,5).

### Comportement en compression

La composition H a été additionnée de 5% de PEG 6000 (Polyéthylèneglycol de masse molaire moyenne de 6000) et comprimée. Les comprimés obtenus, d'un poids de 35,9 g, étaient non friables et disposaient d'une bonne résistance mécanique.

Dans un volume d'eau de 2 litres à une température initiale de 50°C, ils se dissolvent sans agitation en environ 100 minutes grâce à l'effervescence produite.

### Exemples 2 à 6

Les compositions des exemples 2 à 6 selon l'invention ont été préparées et mises sous forme de comprimés d'un poids de 30 g. Les temps de dissolution ont ensuite été mesurés après addition d'un comprimé à un volume d'eau de 3 litres dont la température initiale était de 40°C:

| | Exemple 2 | Exemple 3 | Exemple 4 | Exemple 5 | Exemple 6 |
|---|---|---|---|---|---|
| Na₂CO₃.1,5 H₂O₂ | 47,62 % | 47,62 % | 47,62 % | 47,62 % | 47,62 % |
| TAED | 9,52 % | 9,52 % | 9,52 % | 9,52 % | 9,52 % |
| DDBSNa 85% | 4,76 % | 4,76 % | 4,76 % | 4,76 % | 4,76 % |
| Citrate.3Na 2H₂O | 9,52 % | 9,52 % | 9,52 % | 9,52 % | 9,52 % |
| Na₂SO₄ | 21,73% | 21,73% | 18,87% | 18,87% | 18,87% |
| Azurant optique* | 0,19% | 0,19% | 0,19% | 0,19% | 0,19% |
| PEG 6000 | 4,76 % | 4,76 % | 4,76 % | 4,76 % | 4,76 % |
| SYLOID 244 FP | 1,90 % | / | / | / | / |
| KOLLIDON CL | / | 1,90 % | 4,76 % | / | / |
| POLYPLASDONE XL | / | / | / | 4,76 % | / |
| DISINTEX 200 | / | / | / | / | 1,71% |
| | | | | | |
| Temps de dissolution | > 20 min | 6 min | 4 min | 3 min | 5 min |

| | | | | | |
|---|---|---|---|---|---|
| * Il s'agit du TINOPAL® DMS-X comme dans l'exemple 1 ci-dessus. | | | | | |

On voit que l'addition d'un agent désintégrant adapté comme la Polyvinylpolypyrrolidone (KOLLIDON CL, POLYPLASDONE XL ou DISINTEX 200) diminue de manière significative les temps de dissolution.

## Revendications

1. Utilisation de lavettes pour le nettoyage et/ou pour la désinfection de trayons de vache, lesdites lavettes étant imprégnées d'une solution de nettoyage et/ou de désinfection comprenant :
a) un système alcalin donneur d'oxygène actif contenant au moins un composé libérateur d'oxygène actif ;
b) un activateur du composé libérateur d'oxygène actif ; et
c) un azurant optique.

2. Utilisation d'une solution pour le nettoyage et/ou pour la désinfection d'un faisceau trayeur, ladite solution de nettoyage et/ou de désinfection comprenant :
a) un système alcalin donneur d'oxygène actif contenant au moins un composé libérateur d'oxygène actif ;
b) un activateur du composé libérateur d'oxygène actif ; et
c) un azurant optique.

3. Utilisation selon la revendication 1 ou 2, dans laquelle :
- le composé libérateur d'oxygène actif est présent à raison de 30 à 90%, et de préférence de 40 à 70%, en masse par rapport à la masse totale de l'ensemble des constituants (a) à (c), la teneur en oxygène actif qui en résulte étant comprise entre 4,0 et 13,0%, de préférence entre 5,0 et 10,0% ;
- le rapport de la quantité massique dudit activateur sur la quantité d'oxygène actif en termes massiques est compris entre 0,8 et 3,0, de préférence entre 1,0 et 2,0 ; et
- le rapport de la quantité massique dudit azurant optique sur la quantité d'oxygène actif en termes massiques est compris entre 0,01 et 0,05, de préférence entre 0,015 et 0,025.

4. Utilisation selon l'une quelconque des revendications 1 à 3, dans laquelle le système alcalin donneur d'oxygène actif comprend le percarbonate de sodium, et l'activateur comprend la tétraacétyléthylènediamine (TAED).

5. Utilisation selon l'une quelconque des revendications 1 à 4, dans laquelle la solution comprend en outre au moins un agent choisi dans le groupe constitué par : des agents séquestrants, des agents anti-tartre, des dispersants, des tensio-actifs, des adjuvants de fabrication, des accélérateurs de dissolution, des agents désintégrants, des charges minérales, des colorants, des parfums.

## Claims

1. The use of wash cloths for cleaning and/or disinfecting cow teats, said wash cloths being impregnated with a cleaning and/or disinfecting solution comprising:
a) an active oxygen donor alkaline system containing at least one compound releasing active oxygen;
b) an activator of the compound releasing active oxygen; and
c) an optical brightener.

2. The use of a solution for cleaning and/or disinfecting a milking cluster, said cleaning and/or disinfecting solution comprising:
a) an active oxygen donor alkaline system containing at least one compound releasing active oxygen;
b) an activator of the compound releasing active oxygen; and
c) an optical brightener.

3. The use according to claim 1 or 2, wherein:
- the compound releasing active oxygen is present in an amount from 30 to 90%, and preferably from 40 to 70% by mass based on the total mass of the whole of the constituents (a) to (c), the active oxygen content resulting therefrom being comprised between 4.0 and 13.0%, preferably between 5.0 and 10.0%;
- the ratio of the mass amount of said activator over the amount of active oxygen in terms of mass is comprised between 0.8 and 3.0, preferably between 1.0 and 2.0; and
- the ratio of the mass amount of said optical brightener over the amount of active oxygen in terms of mass is comprised between 0.01 and 0.05, preferably between 0.015 and 0.025.

4. The use according to any of claims 1 to 3, wherein the active oxygen donor alkaline system comprises sodium percarbonate, and the activator comprises tetraacetylethylenediamine (TAED).

5. The use according to any of claims 1 to 4, wherein the solution further comprises at least one agent selected from the group formed by: sequestering agents, anti-scale agents, dispersants, surfactants, manufacturing additives, the dissolution accelerators, disintegrating agents, inorganic fillers, coloring agents, perfumes.

## Patentansprüche

1. Verwendung von Waschlappen für die Reinigung und/oder für die Desinfektion von Kuhzitzen, wobei die Waschlappen mit einer Reinigungs- und/oder Desinfektionslösung getränkt sind, die umfaßt:
a) ein Aktivsauerstoff abgebendes alkalisches System, das wenigstens eine Aktivsauerstoff freisetzende Verbindung enthält,
b) einen Aktivator der Aktivsauerstoff freisetzenden Verbindung und
c) einen optischen Aufheller.

2. Verwendung einer Lösung für die Reinigung und/oder für die Desinfektion eines Melkzeugs, wobei die Reinigungs- und/oder Desinfektionslösung umfaßt:
a) ein Aktivsauerstoff abgebendes alkalisches System, das wenigstens eine Aktivsauerstoff freisetzende Verbindung enthält,
b) einen Aktivator der Aktivsauerstoff freisetzenden Verbindung und
c) einen optischen Aufheller.

3. Verwendung nach Anspruch 1 oder 2, wobei:
- die Aktivsauerstoff freisetzende Verbindung in einer Menge von 30 bis 90 und vorzugsweise von 40 bis 70 Massenprozent bezogen auf die Gesamtmasse aller Bestandteile (a) bis (c) vorliegt, wobei der Aktivsauerstoffgehalt, der sich hieraus ergibt, im Bereich zwischen 4,0 und 13,0 %, vorzugsweise zwischen 5,0 und 10,0 % liegt,
- das Verhältnis der Gewichtsmenge des Aktivators zur Menge an Aktivsauerstoff ausgedrückt in Masse im Bereich zwischen 0,8 und 3,0, vorzugsweise zwischen 1,0 und 2,0 liegt und
- das Verhältnis der Gewichtsmenge des optischen Aufhellers zur Menge an Aktivsauerstoff ausgedrückt in Masse im Bereich zwischen 0,01 und 0,05, vorzugsweise zwischen 0,15 und 0,025 liegt.

4. Verwendung nach einem der Ansprüche 1 bis 3, wobei das Aktivsauerstoff abgebende alkalische System Natriumpercarbonat und der Aktivator Tetraacetylethylendiamin (TAED) umfaßt.

5. Verwendung nach einem der Ansprüche 1 bis 4, wobei die Lösung ferner wenigstens ein Mittel umfaßt, das aus der Gruppe bestehend aus Sequestriermitteln, Kesselsteinverhütungsmitteln, Dispersionsmitteln, Tensiden, Herstellungszusatzmitteln, Lösungsbeschleunigern, Zerfallsmitteln, mineralischen Füllstoffen, Farbstoffen, Duftstoffen ausgewählt ist.
